Europäisches Patentamt

European Patent Office

Office européen des brevets

(11) Publication number: 0 516 846 A1

# EUROPEAN PATENT APPLICATION
## published in accordance with Art. 158(3) EPC

(21) Application number: 90905640.0

(22) Date of filing: 31.03.90

(86) International application number: PCT/JP90/00446

(87) International publication number: WO 90/15660 (27.12.90 90/29)

(51) Int. Cl.5: A61M 5/165, B01D 63/08

(30) Priority: 13.06.89 JP 141383/89

(43) Date of publication of application: 09.12.92 Bulletin 92/50

(84) Designated Contracting States: DE FR GB

(71) Applicant: SAKAMOTO, Atsunobo
3-10-15 Minamiyama, Siroichou
Inba-Gun, Chiba-ken 270-14(JP)
Applicant: SAKAMOTO, Kazuko
3-10-15 Minamiyama, Siroichou
Inba-Gun, Chiba-ken 270-14(JP)

(72) Inventor: SAKAMOTO, Atsunobo
3-10-15 Minamiyama, Siroichou
Inba-Gun, Chiba-ken 270-14(JP)
Inventor: SAKAMOTO, Kazuko
3-10-15 Minamiyama, Siroichou
Inba-Gun, Chiba-ken 270-14(JP)

(74) Representative: Murgatroyd, Susan Elizabeth et al
Baron & Warren 18 South End Kensington
London W8 5BU(GB)

(54) BAG-LIKE FILTER.

(57) This invention relates to a filter for filtrating foreign matters from a fluid. The filter of this invention uses a plastic film bag free from leakage of the fluid as a housing and is produced by fusing a heat-fusible film such as polyethylene to a filter film used generally such as a polysulfone film by heat-sealing, forming a closed bag-like space on one or both sides of the filter film and disposing an inlet and an outlet. Accordingly, even a precision filter or a filter having a big area can be produced easily, completely and more over, economically, in the same way as in bag-making processes. The filter of this invention is most suitable for a precision low-pressure application such as a medical final filtering or blood transfusion, and its usable pressure can be further levated by use of a reinforcing box, or the like.

Fig. 2

[Technical Field]

The present invention relates to a filter for removing foreign matters in a fluid such as liquid or gas. It can also be used as an ordinary filter, but is most suitable for disposable, precision and relatively low pressure applications, such as medical final filter and blood transfusion.

[Background Arts]

Especially in the case of a medical precision filter, it is essential that the filter membrane is perfectly sealed at the periphery. In this case, since no adhesive can be used, the sealing is generally achieved by putting O rings or packings, etc. made of teflon on both sides of the filter membrace, to hold the filter membrace as firmly as possible, and tightening the screws ordinally provided at the edges of the molded plastic housung, or tightening screws provided separately for pressing the housing at the periphery. However, in the case of mass products, the packings, etc. are not used since they raise the cost, and the filter membrane is caught as firmly as possible, while the housing members on both sides of the filter membrane are fused to each other. Partly because of this, it is difficult to perfectly eliminate the clearance at the joint between the housing members and the filter membrane, and for example, even in the case of small Filters of 25 mm in diameter, one hundred percent inspection must be execused after production.

Further more, since a precision filter has smaller pores, the area must be enlarged to secure a certain flow rate, but if the area is larger, it is more difficult and troublesome to perfectly seal the filter membrane at the periphery. Moreover, since a larger molded plastic product requires a higher technique, the cost is raised significantly.

For this reason, it often occurs that a filter lower in precision is used in view of expense. For example, the medical necessity of a final filter for removing foreign matters causing phlebitis in a drug from a drip solution has been pointed out since a long time ago, but even now, only a few hospitals among large hospitals use the best 0.2 $\mu$m filter, and since a less expensive 25 mm dia. filter with 0.2 $\mu$m pores is too small in flow rate, a filter with a larger pore size of 5 $\mu$m is used as a compromise. Most hospitals refrain from using the 0.2 $\mu$m filter since there is no blame against it as a medical failure. A precision filter which is large in filter membrane area, low in cost and disposable has been keenly needed, but any prior art does not allow the easy production of such a filter.

[Disclosure of Invention]

The filter of the present invention uses a plastic film bag which doesn't allow the intended fluid to leak, instead of a molded housing. For example, heat-fusible films of polyethylene, etc. are fused to a filter membrane of generally used polysulfone, etc. by heat sealing, etc., to form a closed baggy space on both sides or one side of the filter membrane, and each of the baggy spaces is provided with an inlet from or outlet to a circuit. If the fluid to be filtered is injected from the inlet to the primary side of the filter, i.e., the baggy space upstream of the filter membrane, the space is filled with the fluid to belly, but since the pressure increases simultaneously, the fluid goes through the filter membrane, while being filtered, to the secondary side, i.e., the baggy space downstream of the filter membrane toward the outlet. It does not occur that the fluid flows from the primary side to the secondary side without being filtered or that the fluid leaks outside. The plastic film bag functions sufficiently as a housing. The sticking between the plastic films and the filter membrane can be achieved simply and perfectly as done in the production of shopping bag. Furthermore, if the bag filter is placed in a reinforcing box, etc., not to be expanded more than required, the working pressure can be raised.

[Brief Description of Drawings]

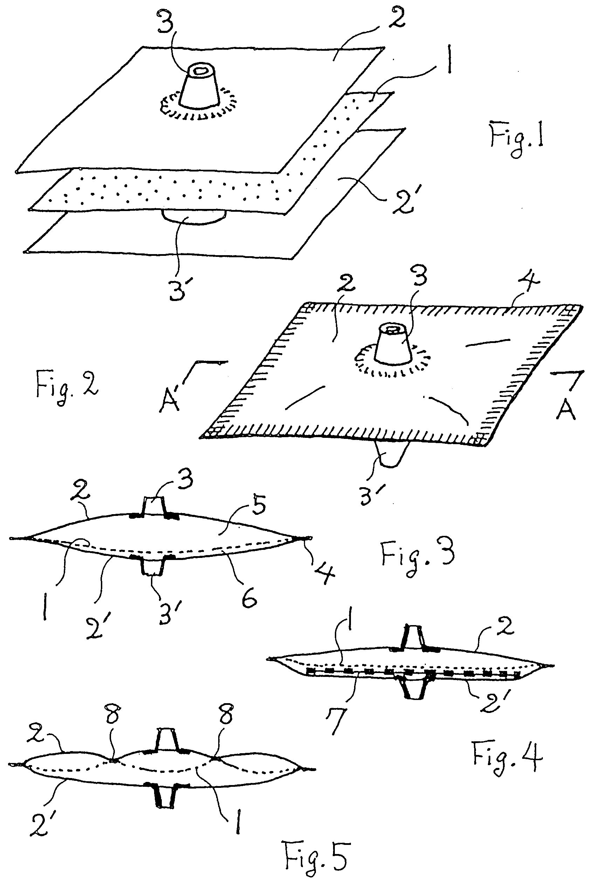

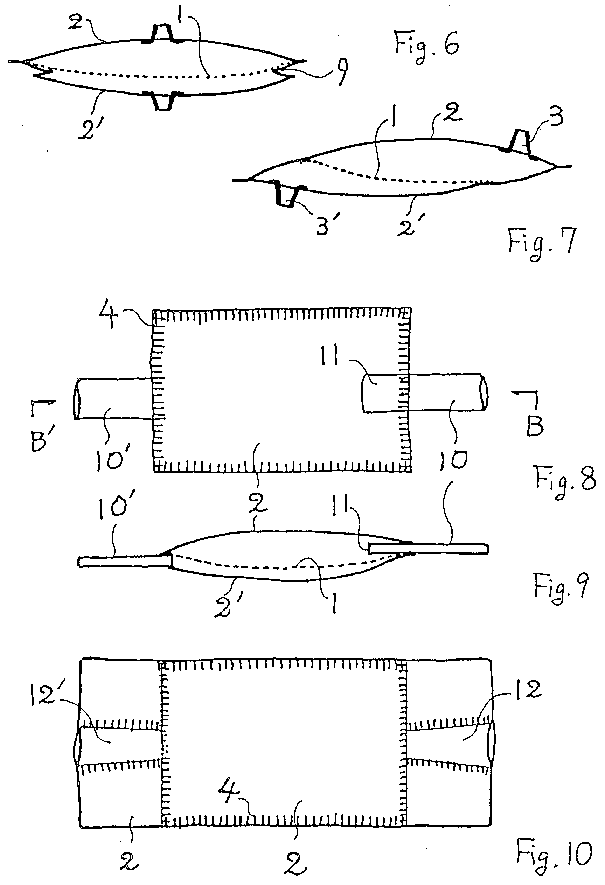

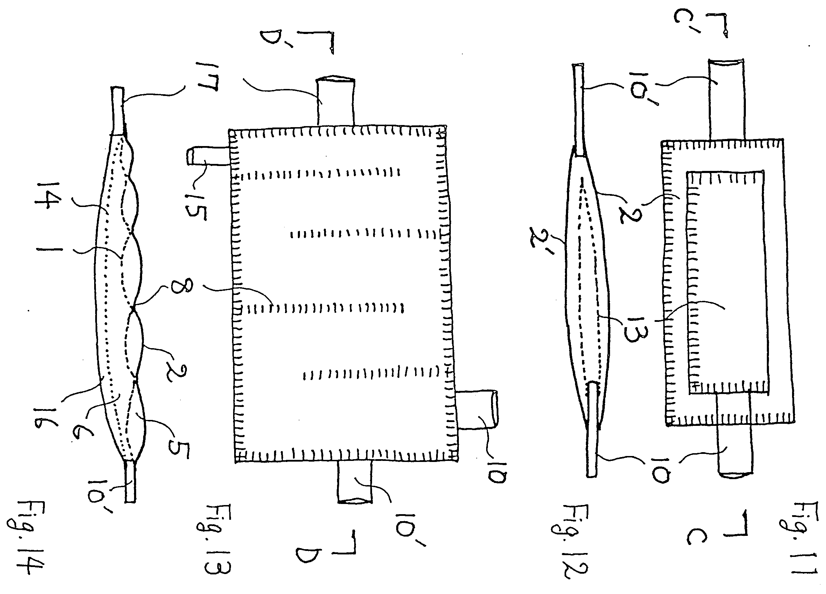

Figs. 1 and 2 are perspective views showing an example of the present invention. Fig. 3 is the AA' sectional view of Fig. 2. Figs. 4 to 7 are sectional views showing its variations. Fig. 8 is a plane view showing another outlet and inlet. Fig. 9 is the BB' sectional view of Fig. 8. Fig. 10 is a plan view showing a further other outlet and inlet. Fig. 11 is a plan view showing another example of filter membrane. Fig. 12 is the CC' sectional view of Fig. 11. Fig. 13. shows a still other example. Fig. 14 is the DD' sectional view of Fig. 13.

[Best Mode for Carring out of the Invention]

Figs. 1 to 3 show an example for illustrating the principle of the present invention. As shown in Fig. 1, polyethylene films 2 and 2' or composite polyethylene films 2 and 2' with a nylon film, etc. stuck to the outside of each of the polyethylene films are lapped on each other, with a flat porous filter membrane 1 made of polysulfone kept between them. Each of the polyethylene films is provided with an inlet 3 or an outlet 3' for the fluid, which is respectively made of the same material as the films, i.e., soft polyethylene, formed by molding to have an overlap portion as thin as, say, 0.5 to 0.8 mm spread around its bottom, and fused to the film to prevent the leak of the fluid, as done with the air inlet of the tire tube for swimming. And as shown in Fig. 2, these films are fused to each other by heating such as heat sealing at the full periphery of the edges 4, to prevent the leak of the fluid. If a molded housing is used, this step of filter membrane sticking is most difficult in view of leak prevention, but in the present device, the sticking step is the same as in the production of shopping bags and so can be simply and perfectly achieved. In this example, if the fusion is imperfect, leak goes outside, and the failure or filtration can be avoided advantageously. If the filter membrane is heat-cut, the possibility that the fluid leaks from an end of the membrane can be prevented.

The AA' sectional view of Fig. 2 is shown in Fig. 3. If the fluid is fed from the inlet 3, the baggy space formed by the polyethylene film 2 and the filter membrane 1, i.e., a primary chamber 5 of the filter bellies like an air bag. At the same time the fluid filtered by the filter membrane 1 goes into the baggy space formed by the filter membrane 1 and the polyethylene film 2', i.e., a secondary chamber 6 of the filter, being collected at the outlet 3'. If the filter membrane is not functionally different between both sides, the fluid can be fed reversely to achieve the same object.

This has no problem at all for low flow rate applications such as a final filter for drip injection, but if highly efficient flow is desired, it is recommended to avoid the state that the secondary chamber is somewhat crushed by the swelling of the primary chamber. Ideas for improving the problem are shown in Figs. 4 and 5 as AA' sectional views as shown in Fig. 3. In Fig. 4, a non-woven fabric or molded product, etc. as a spacer 7 is put in the secondary chamber, to keep the filter membrane 1 away from the secondary film 2'. In Fig. 5' the polyethylene film 2 and the filter membrane 1 are properly fused to each other at portions 8 for example linearly in the primary chamber. In this state, the fluid goes to every portion of the filter membrane, but the filter membrane 1 is not separated so much from the polyethylene film 2, that is, it does not belly so much. Thus, the filter membrane 1 is sufficiently apart from the polyethylene film 2' in the secondary chamber, to raise the flow rate. Furthermore, it is also effective to form a fold 9 in the polyethylene film 2' of the secondary chamber as shown in Fig. 6, to make its area larger than the area of the filter membrane 1. In this case, the filter membrane may be lined with a support screen such as very coarsely woven polyethylene fabric. Moreover, as shown in Fig. 7, both the polyethylene films 2 and 2' can be made larger than the filter membrane 1. In this example, since the inlet 3 and the outlet 3' are arranged to be kept away from the filter membrane 1, the filter membrane 1 which is relatively liable to be flawed can be prevented from being damaged by the hard molded outlet or inlet or by the action of inserting or removing a connector, etc. In addition, the primary chamber may be formed by respective parts of the polyethylene films 2 and 2' as shown in Fig. 7.

For the inlet and outlet, instead of the molded products as described above, polyethylene film tubes 10 and 10' can be fused between the polyethylene film 2 or 2' and the filter membrane 1 at their edges when those films are fused to each other, as shown in Fig. 8 and in Fig. 9 showing the BB' section of Fig. 8. In this case, to secure the passage, the tubes may contain a thin polyester film piece not fused to the polyethylene film tubes or even can be printed unless there is no problem for the fluid. If the tip 11 of the thin tube protrudes into the primary chamber, it acts as a check valve, to let the filter have a direction, and may be useful in some cases. However, if it protrudes in the secondary chamber, flow is prevented, and so it must not protrude in the secondary chamber. Furthermore, as shown in Fig. 10, both the polyethylene films can be used for preparing the inlet and outlet 12 and 12', at the time of fusing them together. In this case, the filter membrane and the polyethylene film on the inlet side or outlet side must be fused to each other at least before the inlet or outlet portion is lapped on the other polyethylene film, or if the three films are lapped and fused together, a material which cannot be fused, for example, a thin polyester film piece must be held for securing the passage. If the inlet 12 and the outlet 12' are tapered like this, and the edges are formed to allow easy fixing, then they can be conveniently easily brought into close contact with other tapered connectors when connected to other circuits.

To make such a filter apparently small in size using an inlet and an outlet, as shown in Fig. 11 and Fig. 12 showing the CC' section of Fig. 11, a filter membrane prepared as a bag 13 instead of a flat film may be positioned between both the polyethylene films 2 and 2'. If the filter membrane is folded double, the width can be a half, and if folded quadruply, one fourth. So, with the same performance kept, the size can be considerably reduced. When the fluid is fed from the side of a tubular port 10', it is recommended to put a spacer in the filter membrane bag 13. Even if the filter membrane bag is prepared by a filter membrane only, the strength is not enough. So, it is recommended to once fuse another polyethylene film to the edges of the filter membrane, for letting the filter membrane have an extended portion around it, and to fold it into a bag, for fusing the double folded and overlapping extended portion together. This method is very effective for increasing the strength not only in the case of filter membrane bag but also in the case of fusion between the filter membran and both the polyethylene films, since when the bag filter is expanded by pressure, the force acting on the fused face is changed from the separating direction to the sliding direction.

In this case, the outside is a bag formed by polyethylene films, an external bag. All the examples shown in Figs. 3 to 7 and 9 can be said to be external bags. There is also an example with a filter membrane held at the joint, but any of the examples does not allow the leak of fluid outside the external bag. And, the inside of each external bag is divided by a filter membrane. These examples can be said to show external bags which are divided into two sections respectively.

When the fluid is, for example, an aqueous solution, though not illustrated, a larger polyethylene film can be stuck around the periphery of the filter membrane 1, with an window opened in it, or the primary side polyethylene film 2 can be partially opened to form a window, and a hydrophobic filter membrane like porous polyethylene tetrafluoride may be fused to provide a function of air vent.

Furthermore, it is also possible to add a filter membrane 14 different in pore size as shown in Fig. 13 and Fig. 14 showing the DD' sectional view of Fig. 13. In this case, it is intended to divide the solid ingredients of the fluid, for example, into large, medium and smoll particles. If the fluid is fed from the inlet 10 of Fig. 13, it gose though the circuit of the primary chamber 5 formed by heat seal lines 8 to reach a narrow outlet 15. In this process, the medium and small particles in the fluid filtered by the filter membrane 1 gose to the secondary chamber 6 sectioned by the second filter 14, to go out of the outlet 10'. In this case, the pressure in the secondary chamber 6 is raised, for example, by narrowing the outlet 10', and so the small particles in the fluid are guided into the second secondary chamber 16 by the second filter membrane 14, to be separated through an outlet 17. Plural filter membranes can be installed in the external polyethylene bag like this.

For medical application, since the presssure required is low, the bag filters described so far can be used without any probrem. However, in order to enhance the pressure resistance, it is possible to let the bag filter be contained in a plastic or other box or bag which is smaller in volume than the expanded volume of the bag filter and cannot be deformed or elongated by the expansion pressure. In this case, the bag filter is pressed against the inner walls of the box or bag, and it does not happen that the walls and sealed portion of the filter are pulled beyond the limit, or that the bag or the sealed portion is broken. Since the filter membrane is pressed tightly, it is necessary to insert an effective spacer on the secondary side, but the pressure resistance of the bag filter can be enhanced to the limit of the strength of the reinforcing box or cover. As the form of the bag filter to be contained in such a box, though not illustrated, if the bag filter contained is formed so that the primary side film of the external bag has a minimum clearance necessary for the fluid to go to every part of the filter membrane, no force acts to make the sealed portion opened at a wide angle. However, for safety, if the sealed portion at the periphery are strongly held, sufficient pressure resistance can be secured at about 5 kg/cm$^2$. The reinforcing box or cover can be used for each bag filter, but if it is designed to allow simple setting and removal of a bag filter, it can be used repeatedly to lower the cost.

In the above examples, baggy spaces are formed on both sides of a filter membrane. However, a bag filter with a baggy space only on one side can be simply manufactured, and has some applications. For example, like a tray as a packaging material, a secondary chamber with an outlet, a spacer and a support screen incorporated can be formed by vacuum molding of an about 0.8 mm thick polyethylene sheat. Though it is difficult to let a filter membrane adhere to a molded product, it can be easily caused to adhere to a film by heat sealing from the film side. So, if a bag filter of primary side is prepared at first, with the fused portion between the filter membrane and the film kept inside the fused portion to be formed later between a molded product and the film, then the intended product can be obtained. The secondary chamber in this case is formed by a film and a molded product like a tray. This example is also a filter of a closed circuit. However, if the circuit is partially opened, the opposite side of the filter membrane is in an air conditioner, culture medium or atmosphere. A filter for sucking a reagent of liquid chromatography is a container. So, the primary side bag filter or secondary side bag filter of the present invention can be used as it is, but with the inlet or outlet formed to suit the intended object.

In the above description, polyethylene films themselves or polyethylene films lined with a nylon film, etc. are used for the external bag which prevents the leak of a fluid, and a porous polysulfone film is used as the filter membrane. These materials are very excellent in view of processing and performance. However, the present invention is not limited to these materials. To suit the respective natures, etc. of fluids, the filter membrane can be selected from porous plastic films including nonwoven fabrics of PP,PVC, vinyl/acrylic copolymer, etc., and the material of the external bag can be the same as that of the filter membrane such as PP,PVC, or ethylene vinyl acetate copolymer, etc. If the material of the external bag is different from that of the filter membrane, heat-fusible plastic films lower in melting point than the filter membrane can be used. The inlet and outlet molded product are of the same material as that of the external bag. As in the case of packaging bags, since composite films laminated with a polyester or aluminium foil, etc. are excellent in view of processing and strength, the description in the claims include also such composite films in addition to single layer films.

Furthermore, the above description refers to fusion only, but unless the fluid is adversely affected, bonding by hot melt or adhesive, etc. can achieve the same effect in the bag filter.

[Industrial Applicability]

Since the housing is not a molded product or sheet metel product, but is formed by plastic films, even a filter with a large area can be prepared very simply at low cost as in the production of plastic bags. Furthermore, the filter can be used as a medical final filter, etc. as it is, and if a reinforcing box or cover is used, it can sufficiently withstand a pressure of about 5 kg/cm$^2$. Plural filter membranes can be contained in the bag. An air vent or check valve can be simly set. The filter membrane can be used for other applications than filtration such as a culture medium. So, the filter of the present invention can be used like an ordinary filter.

Furthermore, since the films of the external bag are transparent, the filter membrane can be observed as in the case of a filter with a transparent molded housing. In addition, since the pressure of the fluid, clogging degree, etc. can be visually observed in reference to the bellying degree of the primary chamber, maintenance is very easy. If the external bag is pressed from above, the amount remaining in the filter can be considerably decreased. If the external bag is directly printed, the matters necessary for the filter can be stated. The filter does not require much space and is light in weight before use, and so can be stored easily. Though depending on the preparation processes, continuous bag filters can be prepared for convenient supply, and a filter with a large area can be formed by winding a long filter membrane. Thus, the filter of the present invention can have many advantages which cannot be achieved by conventional general filters.

## Claims

1. A bag filter, comprising heat-fusible plastic films (hereinafter called the films) of the same material as the filter membrane such as polyethylene or of a different material lower in melting point than the filter membrane, and a plastic filter membrane (hereinafter called the filter membrane) of polysulfone, etc., wherein said filter membrane is lapped as a flat sheet or as a flat bag on the inside walls of the closed bag formed by fusing the lapped films at the full periphery of the lapped plastic films by heat sealing, etc. to prevent the leak of the fluid to be filtered (hereinafter the closed bag is called the external bag), or is held between the lapped filmes fused to form the external bag, with the filter membrane formed as a flat sheet or fused to have a film of the same material as the above films, to have the film around it, or formed as a bag, for dividing the internal space of the external bag into 2 or more spaces; and each of the baggy spaces is provided with an inlet or outlet respectively.

2. A bag filter, comprising heat-fusible plastic films of the same material as the filter membrane such as polyethylene or of a different material lower in melting point than filter membrane, and a plastic filter membrane of polysulfone, etc., wherein said films are lapped on both sides of said filter membrane or said filter membrane fused to a film of the same material as the above films, to have the film around it; they are fused at the full periphery of the lapped plastic films by heat sealing, etc. to form two closed baggy spaces; and each of the baggy spaces is provided with an inlet or outlet respectively.

3. A primary bag filter with an inlet or a secondary bag filter with an outlet, comprising a heat-fusible plastic film of the same material as the filter membrane such as polyethylene or of a different material lower in melting point than the filter membrane, and a plastic filter membrane of polysulfone, etc., wherein said film is lapped on both sides of said filter membrane or said filter membrane fused to a film of the same material as the above film, to have the film around it; they are fused at the full periphery of the lapped plastic film by heat sealing, etc. to form one closed baggy space; and the baggy space is provided with an inlet or outlet.

4. A primary bag filter with an inlet or a secondary bag filter with an outlet, comprising a heat-fusible plastic film of the same material as the filter membrane such as polyethylene or of a different material lower in melting point than the filter membrane, and a plastic filter membrane of polysulfone, etc., wherein said film is lapped on said filter membrane or said filter membrane fused to a film of the material as the above film, to have the film around it; they are fused at the full periphery of the lapped plastic film by heat sealing, etc. to form one closed baggy space; and the baggy space is provided with a filmy or tubular inlet or outlet of the same material as the above film.

5. A primary bag filter, comprising a heat-fusible plastic film of the same material as the filter membrane such as polyethylene or of a different material lower in melting point than the filter membrane, and a molded product made of the same material as the film, and furthermore a plastic filter membrane of polysulfone, etc., wherein said film is lapped on said filter membrane or said filter membrane fused to a film of the same material as the sbove film, to have the film around it; they are fused at the full periphery of the lapped plastic film by heat sealing, etc., to form a bag (primary chamber) closed excluding an inlet provided; the bag with the filter membrane kept inside is lapped on said molded product with devices required for a secondary chamber such as an outlet, a spacer and a supporter; and the film portion protruding from the joint with the filter membrane is fused to the edges of the molded product by heat sealing, etc.

6. A bag filter, comprising plastic films tight against the fluid to be filtered, and a plastic filter membrane of polysulfone, etc., wherein said films are lapped and bonded at the full periphery of the films by an adhesive, etc., to form a bag (external bag), for preventing the leak of the fluid to be filtered, with said filter membrane lapped as a flat sheet or a flat sheet with a film of the same material as the above films fused around it, or as a flat bag, onto the inside walls of the closed bag, or held between the films fused togather, for dividing the internal space of the external bag into two or more spaces; and each of the baggy spaces is provided with an inlet or outlet respectively.

7. A bag filter, according to any of claims 1 to 6, wherein when the fluid is an aqueous solution, etc., the filter membrane with a film of the same material as said plastic films fused around it is further fused to a hydrophobic filter membrane of polyethylene tetrafluoride, etc., to have the filter membrane further around it, or has a window in the film portion to have said hydrophobic filter membrane fused to the window, for forming a composite filter membrane, thereby forming a circuit for releasing the air in the aqueous solution in the passage direction.

8. A bag filter, according to any of claims 1 to 6, wherein when the fluid is an aqueous solution, etc., the external bag has a window opened in the wall of the primary chamber of the filter, to be fused with a hydrophobic filter membrane of polyethylene tetrafluoride, etc., for forming a circuit to release the air in the aqueous solution outside the passage.

9. A reinforcing box or cover of a bag filter according to any of claims 1 to 6, which is a box or bag wiht an internal space not to allow the expansion of the external bag beyond necessity.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

# INTERNATIONAL SEARCH REPORT

International Application No  PCT/JP90/00446

**I. CLASSIFICATION OF SUBJECT MATTER** (if several classification symbols apply, indicate all) 6

According to International Patent Classification (IPC) or to both National Classification and IPC

Int. Cl$^5$  B01D63/08, A61M5/165

**II. FIELDS SEARCHED**

| Minimum Documentation Searched 7 | |
|---|---|
| Classification System | Classification Symbols |
| IPC | B01D63/08, A61M1/34, 5/165 |

| Documentation Searched other than Minimum Documentation to the Extent that such Documents are Included in the Fields Searched 8 |
|---|
| Jitsuyo Shinan Koho            1926 - 1990 |
| Kokai Jitsuyo Shinan Koho      1971 - 1990 |

**III. DOCUMENTS CONSIDERED TO BE RELEVANT** 9

| Category * \ | Citation of Document, 11 with indication, where appropriate, of the relevant passages 12 | Relevant to Claim No. 13 |
|---|---|---|
| X | JP, A, 53-54178 (Johnson & Johnson), 17 May 1978 (17. 05. 78), (Family: none) | 1 - 6 |
| X | JP, A, 54-34178 (Johnson & Johnson), 13 March 1979 (13. 03. 79), (Family: none) | 1 - 8 |
| X | JP, Y2, 54-42156 (Terumo Corp.), 7 December 1979 (07. 12. 79) | 1-6, 9 |
| X | JP, Y2, 54-4121 (Terumo Corp.), 23 February 1979 (23. 02. 79) | 1 - 6 |
| X | JP, Y2, 57-44997 (Terumo Corp.), 4 October 1982 (04. 10. 82) | 1 - 6 |
| X | JP, A, 56-36961 (Nippon Medical Supply K.K.), 10 April 1981 (10. 04. 81), (Family: none) | 1 - 6 |

\* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| June 15, 1990 (15. 06. 90) | July 2, 1990 (02. 07. 90) |
| International Searching Authority | Signature of Authorized Officer |
| Japanese Patent Office | |

Form PCT ISA 210 (second sheet) (January 1985)

International Application No. **PCT/JP90/00446**

| FURTHER INFORMATION CONTINUED FROM THE SECOND SHEET | | |
|---|---|---|
| X | JP, Y2, 58-9610 (Nippon Medical Supply K.K.), 22 February 1983 (22. 02. 83) | 1 - 6 |
| X | JP, A, 56-76206 (Nippon Medical Supply K.K.), 23 June 1981 (23. 06. 81), Scope of claim (2) to (4), Figs. 3 to 5 (Family: none) | 1 - 6 |
| X | JP, B1, 49-4759 (Baxter Laboratories, Inc.), 2 February 1974 (02. 02. 74), (Family: none) | 1 - 6 |

**V.☐ OBSERVATIONS WHERE CERTAIN CLAIMS WERE FOUND UNSEARCHABLE** [1]

This international search report has not been established in respect of certain claims under Article 17(2) (a) for the following reasons:

1.☐ Claim numbers ........ , because they relate to subject matter not required to be searched by this Authority, namely:

2.☐ Claim numbers ........ , because they relate to parts of the international application that do not comply with the prescribed requirements to such an extent that no meaningful international search can be carried out, specifically:

3.☐ Claim numbers ........ , because they are dependent claims and are not drafted in accordance with the second and third sentences of PCT Rule 6.4(a).

**VI.☐ OBSERVATIONS WHERE UNITY OF INVENTION IS LACKING** [2]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

Form PCT/ISA/210 (supplemental sheet (2)) (January 1985)